# EUROPEAN PATENT SPECIFICATION

(11) **EP 0 532 043 B1**
(45) Date of publication and mention of the grant of the patent: **11.06.1997**
(21) Application number: 92115607.1
(22) Date of filing: 11.09.1992
(51) Int. Cl.: C12N 15/66, C12N 15/62, C12N 15/11, C12N 1/21, C07K 14/00

(54) **Factor Xa linker DNA**
Faktor Xa-Linker DNA
Facteur Xa ADN-ligateur

(30) Priority: 13.09.1991 JP 234430/91
(43) Date of publication of application: 17.03.1993
(73) Proprietor: HITACHI, LTD., Chiyoda-ku, Tokyo 101 (JP)
(72) Inventor: Sakamoto, Takeshi, Hatoyamamachi, Hiki-gun, Saitama-ken (JP); Takamoto, Kazunori, Niiza-shi (JP); Senda, Toshiya, Nagaoka-shi (JP); Harada, Yoshinori, Hiki-gun, Saitama-ken (JP)
(74) Representative: Strehl Schübel-Hopf Groening & Partner

(56) References cited:
- EP-A- 0 161 937
- EP-A- 0 327 939
- NATURE vol. 309 , 28 June 1984 , LONDON pages 810 - 812 K. NAGAI AND H. THOGERSEN 'Generation of beta-globulin by sequence-specific proteolysis of a hybrid protein produced in E. coli'
- GENE. vol. 93 , 1990 , AMSTERDAM NL pages 129 - 134 P. MARKMEYER ET AL 'The pAX plasmids: new gene-fusion vectors for sequencing, mutagenesis and expression of proteins in E. coli'

## Description

### FIELD OF THE INVENTION

The present invention relates to a gene corresponding to recognition amino acid sequence of blood coagulation factor Xa having a restriction protease activity, a fusion gene bearing the same, an expression vector and a process for preparing Escherichia coli bearing the expression vector.

### RELATED ART STATEMENT

In genetic engineering technique for expressing a protein by transferring a foreign gene to a host cell such as Escherichia coli, it is effective to insert a desired gene in the middle of structural gene of the existing expression vector having a high efficiency thereby to obtain a fusion protein in large quantities [for example, Maniatis et al., Molecular Cloning: A Laboratory Manual, second edition, pages 17.3-17.9, Cold Spring Harbor Laboratory Press, 1989]. In this case, for isolation of a desired mature protein, various techniques have been developed to specifically cleave the fusion site alone in the fusion protein [for example, D.V. Geoddel, Methods in Enzymology, 185, 129-143 (1990)]. Among them, attention has been focused on a technique utilizing blood coagulation factor Xa capable of specifically recognizing amino acid sequence Ile-Glu-Gly-Arg and having a protease activity which digests C-terminal side of Arg.

A representative process for obtaining a desired mature protein comprises inserting DNA linker containing the nucleotide sequence corresponding to factor Xa recognition amino acid sequence (hereafter abbreviated as Xa linker DNA) into restriction enzyme Bam HI cleavage site in structural gene of the existing expression vector, inserting a desired gene with a blunt 5' terminus into restriction enzyme Stu I cleavage site in the linker and treating the expressed fusion protein with factor Xa (Kiyoshi Nagai et al., Japanese Patent Application Laid-Open No. 61-135591). In addition to the process, there are disclosed a process which comprises fusing Xa linker DNA with human B cell differentiation gene at the upstream thereof (Yoshihiro Asagoe et al., Japanese Patent Application Laid-Open No. 2-2353), a process which comprises fusing Xa linker DVA with human growth hormone gene at the upstream thereof (G. Grandi et al., Japanese Patent Application Laid-Open No. 2-2386), a process which comprises ligating a gene encoding several glycine residues before and after Xa linker DNA (A. Pluckthun et al., Japanese Patent Application Laid-Open No. 2-5881).

### SUMMARY OF THE INVENTION

Upon fusing Xa linker DNA and a desired foreign gene, there are two problems. One is to effect fusion so as to adjust a reading frame of codon and another is to form a mature foreign protein upon digestion of a fusion protein with factor Xa. Among the prior art processes described above, Japanese Patent Application Laid-Open No. 61-135591 deals with a general method for fusion with a foreign gene. In this case, the fused site is recognition sequence AGGCCT of restriction enzyme Stu I present in the inside of Xa linker DNA. By digestion with Stu I, Arg codon AGG forms blunt 3' end at the upstream site of fusion. Therefore, in order to attain the two problems described above, it is necessary to prepare a foreign gene in such a manner that the 5' end nucleotide be rendered blunt to be the first letter of the first codon.

In general, however, it is difficult to render the 5' end of a foreign gene to be fused blunt so as to correspond to the first letter of codon. Its probability is one-third. In other cases, it is impossible to use a vector shown in the representative process described above.

An object of the present invention is to provide linker DNA for an insert gene rendered blunt to form a 5' end added with one nucleotide to reading frame of codon, which linker DNA is capable of ligating DNA encoding factor Xa recognition sequence at the upstream of said gene with a complete suite in reading frame of codon.

Another object of the present invention is to provide a process for preparing a fusion gene which comprises ligating a desired foreign gene with an optional structural gene having a specific restriction enzyme site through Xa linker DNA according to the invention.

A further object of the present invention is to provide a process for preparing a fusion gene having ligated with a desired foreign gene in tandem through Xa linker DNA according to the invention.

### BRIEF DESCRIPTION OF THE DRAWINGS

Fig. 1 shows nucleotide sequence of Xa linker DNA and procedures for preparing an expression vector fused with the linker which is an embodiment of the present invention.

Fig. 2 shows procedures for preparing an expression vector bearing Xa linker DNA with which the fourth fragment gene of octopus rhodopsin is fused as a foreign gene, which is an embodiment of the present invention.

Fig. 3 shows procedures for preparing 5' end of a foreign gene to be fused with an expression vector bearing Xa linker DNA.

Fig. 4 shows nucleotide sequence of Xa linker DNA used for tandem ligation of genes and procedures for preparing an expression vector fused with the linker which is an embodiment of the present invention.

Fig. 5 shows procedures for preparing an expression vector bearing Xa linker DNA with which the two fourth fragment genes of octopus rhodopsin are fused as foreign genes in tandem, which is an embodiment of the present invention.

### DESCRIPTION OF THE PREFERRED EMBODIMENTS

According to the present invention, the factor Xa linker DNA contains the recognition sequence TCGCGA of restriction enzyme Nru I and is cleavable with Nru I to give the blunt fused upstream portion containing the first 2 letters (CG) of the codon for Arg corresponding to the fourth segment of recognition amino acid sequence of factor Xa. Therefore, the 5' end of a foreign gene to be fused is ligated with the upstream portion in such a way that the 5' end is added to the site toward the 5' side by one nucleotide than the reading frame of codon, whereby a fused gene is provided without breaking up the reading frame. Since the first and second letters of Arg codon are CG in this case, a nucleotide corresponding to the third letter may be any one of A, C, G and T in order for this codon to encode Arg. Therefore, a nucleotide added to the 5' end of a foreign gene to be fused is also optional.

In general, the probability that the 5' end of a foreign gene is rendered blunt in such a way that one more extra nucleotide is added to the reading frame is one-third. By progress of genetic engineering in these days, however, it has been increasing to chemically synthesize a desired gene wholly or partly. In most cases, a recognition site of restriction enzyme is provided at 5' end of a gene. Therefore, a process for modifying a foreign gene having a restriction site at the 5' end thereof in such a manner that the present invention can utilize it is explained by referring to its specific embodiments.

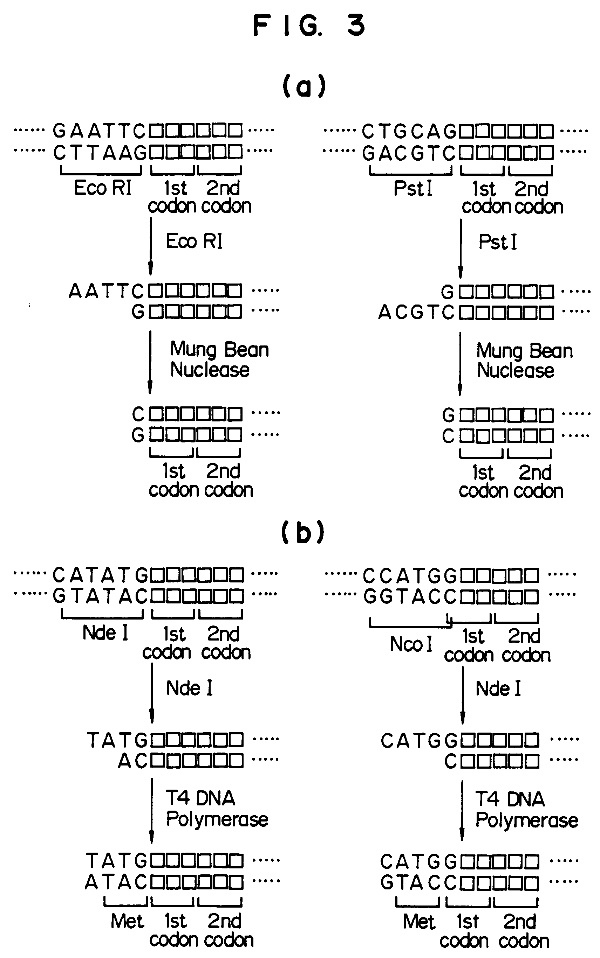

Firstly, where a restriction enzyme capable of recognizing 6 bases represented by Bam HI, Eco RI, Hind III, Pst I, Sac I, etc. immediately before the first codon of a foreign gene to form 4 nucleotide staggered end after cleavage is present (Fig. 3a), the staggered end is removed by Mung Bean nuclease or S1 nuclease after cleave with the restriction enzyme, where one nucleotide remains just before the first codon of a foreign gene in an added state and this nucleotide can construct the third letter of Arg.

Next, where 5' end of a foreign gene has restriction enzyme Nde I recognition sequence CATATG or Nco I recognition sequence CCATGG containing initiation codon (Fig. 3b), the staggered end is filled up with T4 DNA polymerase after cleave with the restriction enzyme. The 5' end-blunt gene added with one nucleotide immediately before initiation codon can thus be prepared. In this case, factor Xa is cleaved after expression of a fusion protein to obtain a foreign protein added with Met at the N terminus. The present invention is applicable not only to these embodiments but also to genes having many kinds of restriction enzymes at 5' end.

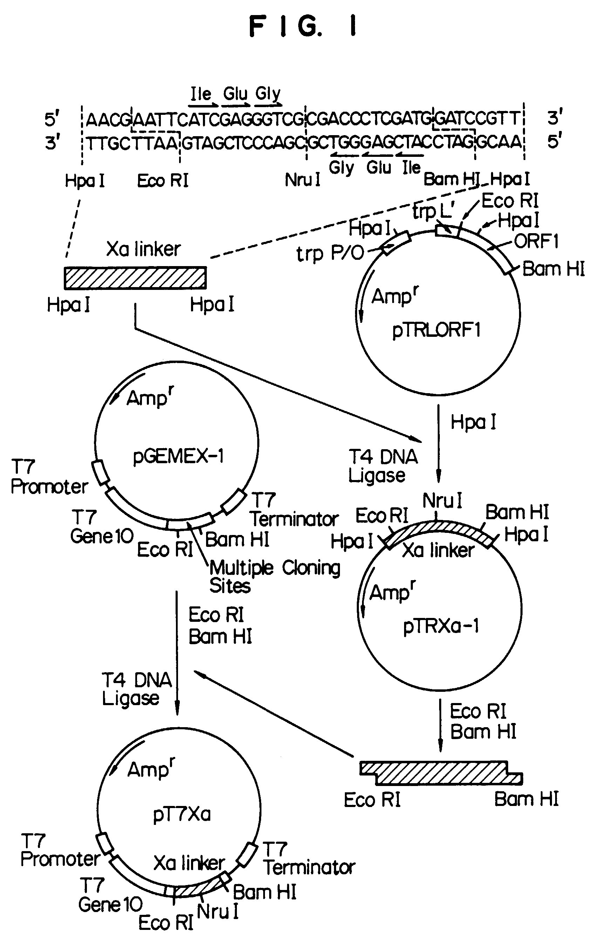

In order to give information on factor Xa recognition sequence to both strands of a DNA, the present invention further provides Xa linker DNA containing inverted repeats (palindrome) sequence of 22 nucleotide pairs centering around Nru I recognition site as shown in Fig. 1 in the inside thereof. According to the present invention, appropriate restriction enzyme recognition sequences are provided on both sides of factor Xa linker DNA, whereby the factor Xa linker DNA can be inserted into the inside of an optional structural gene having specific restriction sites.

For example, in Xa linker DNA shown in Fig. 1 AACGAATTC ATC GAG GGT CGC GACCCTCGAT GGATCCGTT (which is claimed in claim 5), recognition sequences by Eco RI and Bam HI are added to the outside of Xa recognition sequence, and the linker can be inserted into an optional structural gene containing Eco RI and Bam HI recognition sites. In this case, the order of Eco RI and Bam HI recognition sites in the structural gene does not matter because of palindromic nature of the linker. Further in Xa linker: AACGAATTCA TCGAGGGTCG CGACCCTCGA TGAATTCGTT (which is claimed in claim 6) with a structure having Eco RI recognition sequence at both sides outside Xa recognition sequence, the linker can be inserted into an optional structural gene containing Eco RI recognition site but containing no Bam HI recognition sequence.

According to the present invention, two or more foreign genes are ligated in tandem via Xa linker DNA to prepare a fusion gene, in which a fusion protein containing a plurality of foreign proteins can be prepared by expression of the gene. Therefore, productivity of foreign protein recovered by cleavage with factor Xa can be improved.

According to the present invention, a vector bearing a fusion gene of two structural genes via Xa linker DNA can be obtained by ligating factor Xa linker DNA in the structural gene of an expression vector using restriction enzyme, etc. to make up reading frame of codon and then ligating a desired foreign gene or a plurality of foreign genes via Xa linker DNA with Xa linker DNA at restriction enzyme Nru I recognition side to make up reading frame of codon. Further by incubating Escherichia coli transformed with the expression vector, a fusion protein corresponding to the fusion gene is produced. Furthermore, a mature foreign gene products can be obtained by recovering the fusion protein and treating the protein with factor Xa having restriction protease activity.

Hereafter specific embodiments of the present invention are explained below but the present invention is not construed to be limited thereto. Restriction enzymes used in the embodiments are those manufactured by Takara Shuzo Co., Ltd. Media, buffer solutions, etc. were prepared according to the Maniatis et al. manual described above (Molecular Cloning: A Laboratory Manual, second edition, Cold Spring Harbor Laboratory Press, 1989).

### EMBODIMENT 1

### Synthesis and cloning of blood coagulation factor Xa linker DNA:

Using DNA synthesizer (manufactured by Applied Biosystems Co., Ltd., Model 380B), two oligonucleotides of 40 base length shown in Fig. 1 were synthesized by phosphoramidite method. Ammonia water was added to the oligonucleotides. The mixture was allowed to stand at 55°C for 12 hours to remove protective groups followed by purification using OPC (oligonucleotide purification cartridge) column. After the solvent was distilled off by centrifugation under reduced pressure, the solidified two oligonucleotides were dissolved in 100 µl of TE buffer solution, respectively. Absorbance was measured at 260 nm to assay concentration. Then, 0.1 nanomol each of the thus purified and assayed oligonucleotides was taken and 5' end phosphorylation was performed at 37°C for an hour in 50 mM Tris hydrochloride (pH 8.0), 10 mM MgCl₂, 10 mM 2-mercaptoethanol, 1 mM ATP and 20 U of T4 polynucleotide kinase (manufactured by Toyobo). Then the two phosphorylated products were mixed with each other and the mixture was treated with phenol and precipitated with ethanol. The recovered DNA was dissolved in 10 µl of ligation buffer solution (100 mM Tris hydrochloride (pH 7.6), 6.5 mM MgCl₂). After allowing to stand at 70°C for 15 minutes, the solution was gradually annealed to room temperature. By the foregoing procedures, about 10 ng/µl of factor Xa linker DNA was obtained.

Next, a process for preparing plasmid containing the linker DNA described above is explained with reference to Fig. 1. Firstly, plasmid vector pTRLORF1 disclosed in Japanese Patent Application Laid-Open No. 3-123486 was cleaved with restriction enzyme Hpa I having recognition site in trip P/O and gene ORF1 as shown in Fig. 1. E. coli HB101 [pTRLORF1] bearing vector pTRLORF1 was deposited in Fermentation Research Institute of the Agency of Industrial Science on October 5, 1989 and given Accession No. FERM P-11043, and then transferred to an international deposition under the Budapest Treaty and given Accession No. FERM BP-3971.

Then, in order to prevent self ligation of the vector, 5' end dephosphorylation of Hpa I fragment having 4.58 kb was effected using alkaline phosphatase (manufactured by Toyobo) of Escherichia coli. Thereafter the amount corresponding to 100 ng was taken and ligated with 30 ng of the factor Xa linker DNA described above at 16°C for 8 hours using ligation kit (manufactured by Takara Shuzo Co., Ltd.). Using 10 µl of the reaction solution, Escherichia coli competent cell DH5 (manufactured by Toyobo) was transformed to obtain recombinant Escherichia coli DH5 [pTRXa-1] and DH5 [pTRXa-2]. Herein pTRXa-2 is plasmid in which direction of [Hpa I-Hpa I] insert fragment of pTRXa-1 is reversed. This E. coli DH5 [pTRXa-1] was deposited in Fermentation Research Institute of the Agency of Industrial Science on September 10, 1991 and given Accession No. FERM P-12501, and then transferred to an international deposition under the Budapest Treaty and given Accession No. FERM BP-3936.

The linker DNA shown in Fig. 1 is so designed that the ends become Hpa I cleavage ends. Therefore, recombinant vector pTRXa-1 and pTRXa-2 prepared there can be again cleaved with Hpa I and are characterized by cloning the Xa linker DNA again to another optional vector capable of forming blunt ends.

Subsequently, insertion of the Xa linker DNA described above into the existing expression vector pGEMEX-1 (Promega Co.) is described below. pGEMEX-1 contains T7 phage-derived promoter and gene 10 encoding T7 major head protein and also has multiple cloning sites (MCS) of restriction enzyme in the gene. This vector was cleaved with Eco RI and Bam HI in MCS and the termini were dephosphorylated using alkaline phosphatase. On the other hand, vector pTRXa-1 was cleaved with Eco RI and Bam HI to recover Xa linker DNA fragment. The Xa linker DNA fragment was ligated with the vector described above using a ligation kit. Using the reaction solution, Escherichia coli competent cell DH5 was transformed to obtain recombinant E. coli DH5 [pT7Xa].

### EMBODIMENT 2

### Construction of fusion gene between T7 gene 10 and octopus rhodopsin fragment gene via factor Xa linker DNA:

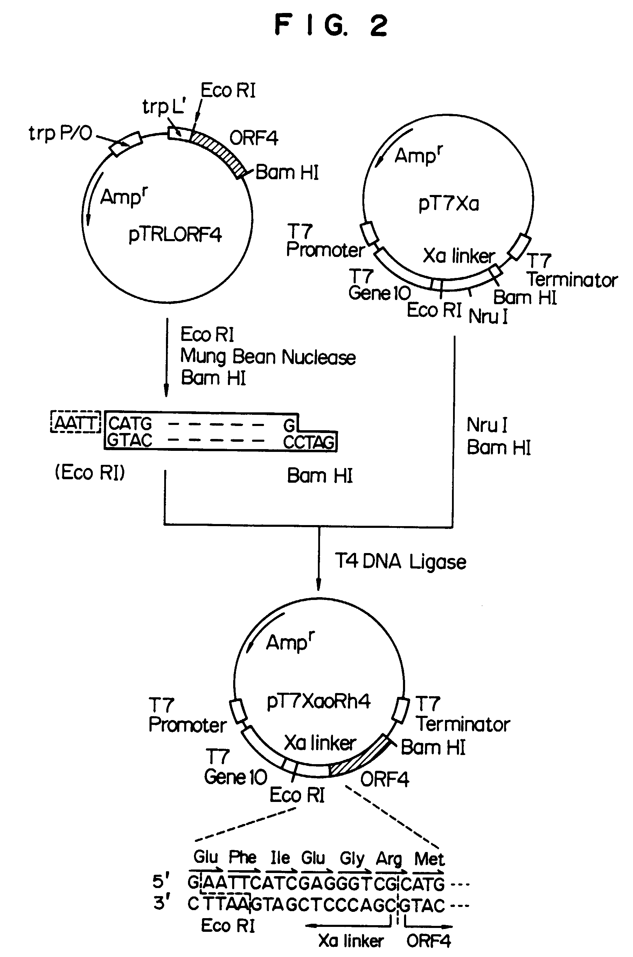

By inserting a desired foreign gene into Nru I recognition site in the Xa linker portion in expression vector pT7Xa prepared in EMBODIMENT 1, the fusion gene of T7 gene 10 and the foreign gene via factor Xa linker DNA can be constructed. As an embodiment, a process for fusing the 4th fragment gene which is a partial gene of octopus rhodopsin gene disclosed in Japanese Patent Application Laid-Open No. 3-123486 with the expression vector is described with reference to Fig. 2.

Firstly, vector pTRLORF4 containing octopus rhodopsin 4th fragment gene was cleaved with Eco RI. Then 5' staggered portion (AATT) was removed using Mung Bean nuclease (manufactured by Takara Shuzo Co., Ltd.) to render 5' end blunt. After Mung Bean nuclease was removed by treatment with phenol, cleavage was effected with Bam HI to recover ORF4 portion of the 4th fragment gene. On the other hand, expression vector pT7Xa was cleaved with Nru I and Bam HI followed by dephosphorylation of 5' end using an alkaline phosphatase. Both were ligated using a ligation kit. E. coli DH5 was transformed with the reaction solution to give recombinant E. coli DH [pT7XaoRh4]. This E. coli DH5 [pT7XaoRh4] was deposited in Fermentation Research Institute of the Agency of Industrial Science on September 10, 1991 and given Accession No. FERM P-12500, and then transferred to an international deposition under the Budapest Treaty and given Accession No. FERM BP-3935.

As shown in Fig. 2, Xa linker 3' end in vector pT7Xa cleaved with Nru I corresponds to the second letter of Arg codon. On the other hand, the 5' end of ORF4 to be fused was rendered blunt in such a way that one nucleotide was added to reading frame of codon. By ligating the ends with each other, the fusion gene was constructed to make up the reading frame.

### EMBODIMENT 3

### Expression of fusion gene and production of mature protein using factor Xa:

Firstly, host E. coli JM109 (DE3) for expression was transformed with expression vector pT7XaoRh4 prepared in EMBODIMENT 2. Recombinant E. coli JM109 (DE3) [pT7XaoRh4] which had been converted into a single colony was cultured at 37°C overnight in 10 ml of NZCYM medium supplemented with 50 µg/ml of ampicillin. Then 0.5 ml of the thus obtained culture broth was cultured at 37°C in 100 ml of NZCYM medium supplemented with 50 µg/ml of ampicillin. At the time when absorbance at 600 nm showed 0.9, isopropyl-β-D-thiogalactopyranoside (IPTG, manufactured by Wako Pure Chemical Industry Co., Ltd.) was added to the system in a final concentration of 1 mM followed by incubation overnight. After completion of the incubation, the culture broth was centrifuged and the cells were collected. The cells were resuspended in 10 ml of 10 mM sodium phosphate buffer (pH 7.0) containing 150 mM sodium chloride and 0.02% (W/V) sodium azide and the cells were homogenized using a French press. The homogenate was centrifuged at 3,000 G for 15 minutes to separate into the supernatant and the precipitates (Fraction 1). The supernatant was further centrifuged at 180,000 G for one hour to separate into the supernatant (Fraction 2) and the precipitates (Fraction 3). Fractions 1, 2 and 3 correspond to inclusion body, cytoplasmic protein and membrane protein, respectively. These fractions were subjected to sampling, respectively, followed by SDS-polyacrylamide gel electrophoresis (SDS-PAGE) having a gel concentration of 16.5%. A band having a molecular weight of approximately 40,000 was detected in Fraction 1. It was thus revealed that the fusion protein of T7 major head protein and octopus rhodopsin 4th fragment protein via Xa recognition sequence was accumulated as inclusion bodies in E. coli.

After rinsing 5 times with 1 mM EDTA solution containing 0.5% Triton X-100, Fraction 1 was dissolved in 5 ml of 50 mM Tris hydrochloride buffer (pH 8.0) containing 8 M urea, 1 mM EDTA and 1 mM dithiothreitol. The solution was then dialyzed to 100 mM sodium chloride and 50 mM Tris hydrochloride buffer solution (pH 8.0) for 18 hours. The dialysate was centrifuged but no precipitate was noted. The crude solution of this fusion protein was determined to have a concentration of about 10 mg/ml by Bradford assay method (Anal. Biochem., 72, 248 (1976)).

After 8.5 µl of a reaction buffer solution (100 mM NaCl, 50 mM Tris hydrochloride (pH 8.0) and 1 mM CaCl₂) was added to 1 µl of the solution of the crude fusion protein, 0.5 µl of restriction protease factor Xa (1 µg/µl, manufactured by Takara Shuzo Co., Ltd.) was added to the mixture followed by reacting them at 25°C for 24 hours. The reaction solution was applied to SDS-PAGE having a gel concentration of 16.5% described above. Most of the cleavage reaction proceeded so that a band corresponding to mature octopus rhodopsin 4th fragment was detected around 7 kilo daltons. This band was subjected to blotting onto Clear Blot-2 Membrane (manufactured by Ato K.K.) and it was confirmed that amino acid sequence of N-terminal 10 residues coincided with mature octopus rhodopsin 4th fragment protein.

### EMBODIMENT 4

### Construction and expression of fusion gene with which octopus rhodopsin fragment gene was ligated in tandem via factor Xa linker DNA:

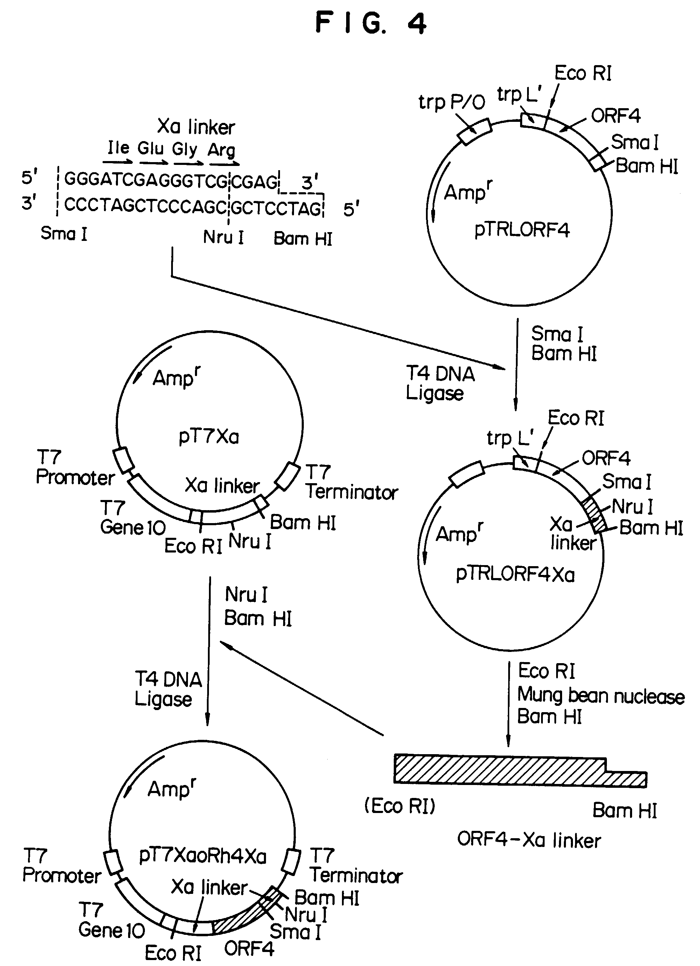

With reference to Fig. 4, a process for preparing factor Xa linker DNA for ligating a foreign gene in tandem is described below. Firstly, using the procedures shown in EMBODIMENT 1, oligonucleotides having lengths of 18 and 22 nucleotides shown in Fig. 4 were synthesized, purified and annealed to give factor Xa linker DNA. Subsequently, the factor Xa linker DNA described above was inserted into vector pTRLORF4 at restriction enzymes Sma I and Bam HI sites located immediately before and after termination codon of the 4th fragment gene of octopus rhodopsin, respectively, to give recombinant vector pTRLORF4Xa (cf. EMBODIMENT 1). Then, using the procedures shown in EMBODIMENT 2, the ORF4-Xa linker portion was recovered from vector pTRLORF4Xa in such a state that the 5' staggered portion was removed. The ORF4-Xa linker portion was inserted into Nru I-Bam HI site of vector pT7Xa prepared in EMBODIMENT 1 to give expression vector pT7XaoRh4Xa. This vector is equivalent to vector pT7XaoRh4 gene, which had been prepared in EMBODIMENT 2, inserted with Xa linker at the 3' end thereof.

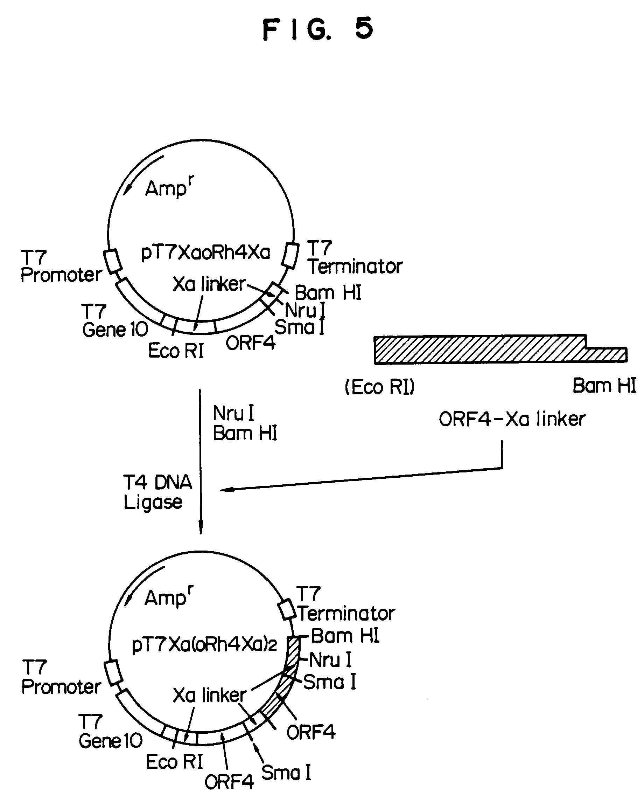

Then following Fig. 5, a process for preparing an expression vector with which 2 foreign genes were ligated in tandem is described. ORF4-Xa linker portion obtained by the procedures described above was inserted into Nru I-Bam HI portion of vector pT7XaoRh4Xa to give expression vector pT7Xa(oRh4Xa)2. This vector contains a fusion gene obtained by ligating T7 gene 10 and two octopus rhodopsin 4th genes (ORF4) via Xa linker DNA. By repeating the procedures for inserting a vector into this ORF4-Xa linker portion, a fusion gene containing 3 or more ORF4 can easily be prepared.

In order to verify expression of the fusion gene, host E. coli JM109 (DE3) was transformed with expression vector pT7Xa(oRh4Xa)2. Furthermore, the fusion protein was expressed and recovered following the procedures shown in EMBODIMENT 3. As the result, a band having a molecular weight of about 50,000 was detected by SDS-PAGE and octopus rhodopsin 4th fragment protein of mature type at the N-terminus was verified by treatment with factor Xa. Since Ile-Glu-Gly-Arg which was factor Xa recognition sequence was added to the C-terminus of this protein, it was confirmed that its electrophoretic distance was shifted toward somewhat higher molecular weight side than the mature octopus rhodopsin 4th fragment protein obtained in EMBODIMENT 3.

The present invention has enabled to fuse a foreign gene with an expression vector bearing a promoter/structural gene having a high expression efficiency into the structural gene via the Xa linker DNA. In most cases, a foreign gene, especially a gene of higher animal and plant does not generally achieve a high expression efficiency with a vector directly ligated at the downstream of promoter/Shine-Dalgarno (SD) sequence. Therefore, the process of the present invention for producing a mature protein which comprises preparing a fusion gene according to the present invention, expressing the fusion gene in E. coli as a fusion protein in large quantities, cleaving the fusion protein with factor Xa greatly contributes to effectively extending applicable subjects of genetic engineering to a wider range.

## Claims

1. A DNA sequence of 14 nucleotide pairs encoding the blood coagulation factor Xa recognition amino acid sequence: Ile-Glu-Gly-Arg wherein the nucleotide sequence coding for Gly-Arg is GGTCGC and the nucleotide sequence GA is added immediately thereafter to form restriction enzyme NruI recognition site TCGCGA.

2. A palindrome DNA sequence of 22 nucleotide pairs consisting of the first 11 nucleotide pairs from the 5' end of the sequence of claim 1 and, immediately thereafter, the inverted 11 nucleotide pair sequence thereof.

3. A DNA sequence containing a DNA sequence of claim 1 and having restriction enzyme recognition sequences or restriction enzyme cleavage ends at both sides of the sequence of claim 1.

4. A DNA sequence containing a DNA sequence of claim 2 and having restriction enzyme recognition sequences or restriction enzyme cleavage ends at both sides of the sequence of claim 2.

5. A DNA sequence according to claim 4, which has a base sequence as described below (SEQ ID NO:1):

AACGAATTC ATC GAG GGT CGC GACCCTCGAT GGATCCGTT

6. A DNA sequence according to claim 4, which has a base sequence as described below (SEQ ID NO:2):

AACGAATTC ATC GAG GGT CGC GACCCTCGAT GAATTCGTT

7. A process for preparing a DNA sequence containing a fusion gene which comprises inserting factor Xa linker DNA according to any one of claims 3 to 6 into the structural gene of an expression vector and ligating a new foreign gene with the restriction enzyme NruI cleavage site of said linker DNA.

8. A process for preparing a DNA sequence containing a fusion gene according to claim 7, wherein a fusion gene obtained by ligating at least two foreign genes in tandem via factor Xa linker DNA is used as said new foreign gene.

9. An expression vector bearing a fusion gene in which a factor Xa linker DNA according to any one of claims 3 to 6 is contained in the structural gene of the expression vector.

10. An expression vector according to claim 9 wherein the factor Xa linker DNA is inserted in the gene 10 of a vector containing the T7 phage-derived promoter and the T7 gene 10 and a new foreign gene is ligated with the restriction enzyme NruI cleavage site of said linker DNA.

11. An expression vector according to claim 10, wherein a fusion gene obtainable by ligating at least two foreign genes in tandem via factor Xa linker DNA is used as said new foreign gene.

12. An expression vector according to any one of claims 9 to 11, wherein said foreign gene is octopus rhodopsin or a fragment thereof.

13. A host organism containing an expression vector according to any one of claims 9 to 12.

14. A host organism according to claim 13, wherein said organism is E. coli.

## Patentansprüche

1. DNA-Sequenz aus 14 Nucleotidpaaren, die die Blutgerinnungsfaktor Xa-Aminosäureerkennungssequenz codiert: Ile-Glu-Gly-Arg wobei die Gly-Arg codierende Nucleotidsequenz GGTCGC ist und die Nucleotidsequenz GA unmittelbar dahinter angefügt ist, wobei die Restriktionsenzym NruI-Erkennungsstelle TCGCGA entsteht.

2. Palindrome DNA-Sequenz aus 22 Nucleotidpaaren, die aus den ersten 11 Nucleotidpaaren vom 5'-Ende der Sequenz gemäß Anspruch 1 und aus deren unmittelbar dahinterliegenden invertierten Sequenz aus 11 Nucleotidpaaren besteht.

3. DNA-Sequenz, die eine DNA-Sequenz gemäß Anspruch 1 und Restriktionsenzymerkennungssequenzen oder Restriktionsenzymschnittstellen an beiden Seiten der Sequenz gemäß Anspruch 1 enthält.

4. DNA-Sequenz, die eine DNA-Sequenz gemäß Anspruch 2 und Restriktionsenzymerkennungsstellen oder Restriktionsenzymschnittstellen an beiden Seiten der Sequenz gemäß Anspruch 2 enthält.

5. DNA-Sequenz gemäß Anspruch 4, die eine wie nachstehend beschriebene Basensequenz enthält (SEQ ID NO:1):

AACGAATTC ATC GAG GGT CGC GACCCTCGAT GGATCCGTT

6. DNA-Sequenz gemäß Anspruch 4, die eine wie nachstehend beschriebene Basensequenz (SEQ ID NO:2) enthält:

AACGAATTC ATC GAG GGT CGC GACCCTCGAT GAATTCGTT

7. Verfahren zur Herstellung einer ein Fusionsgen enthaltenden DNA-Sequenz, welches das Einsetzen einer Faktor Xa-Linker-DNA gemäß einem der Ansprüche 3 bis 6 in das Strukturgen eines Expressionsvektors und das Verbinden eines neuen Fremdgens mit der Restriktionsenzym Nrul-Schnittstelle der Linker-DNA umfaßt.

8. Verfahren zur Herstellung einer ein Fusionsgen enthaltenden DNA-Sequenz gemäß Anspruch 7, wobei ein Fusionsgen, das durch Verbinden von mindestens zwei Fremdgenen in Tandemanordnung über die Faktor Xa-Linker-DNA erhalten wurde, als das neue Fremdgen verwendet wird.

9. Expressionsvektor, der ein Fusionsgen trägt, in welchem eine Faktor Xa-Linker-DNA gemäß einem der Ansprüche 3 bis 6 in dem Strukturgen des Expressionsvektors enthalten ist.

10. Expressionsvektor gemäß Anspruch 9, worin die Faktor Xa-Linker-DNA in das Gen 10 eines Vektors, der den vom Phagen T7 abgeleiteten Promotor und das T7-Gen 10 enthält, eingefügt ist, und ein neues Fremdgen mit der Restriktionsenzym Nrul-Schnittstelle der Linker-DNA verbunden ist.

11. Expressionsvektor gemäß Anspruch 10, worin ein Fusionsgen, das durch Verbinden von mindestens zwei Fremdgenen in Tandemanordnung über eine Faktor Xa-Linker-DNA erhalten werden kann, als das neue Fremdgen verwendet wird.

12. Expressionsvektor gemäß einem der Ansprüche 9 bis 11, worin das Fremdgen Octopus-Rhodopsin oder ein Fragment desselben ist.

13. Wirtsorganismus, der einen Expressionsvektor gemäß einem der Ansprüche 9 bis 12 enthält.

14. Wirtsorganismus gemäß Anspruch 13, wobei der Organismus E. coli ist.

## Revendications

1. Séquence d'ADN de 14 paires de nucléotides codant pour la séquence d'acides aminés de reconnaissance du facteur Xa de coagulation du sang : Ile-Glu-Gly-Arg dans laquelle la séquence nucléotidique codant pour Gly-Arg est GGTCGC et la séquence nucléotidique GA est ajoutée immédiatement après pour former le site de reconnaissance TCGCGA de l'enzyme de restriction NruI.

2. Séquence d'ADN palindromique de 22 paires de nucléotides constituée des 11 premières paires de nucléotides de l'extrémité 5' de la séquence de la revendication 1 et, immédiatement après, la séquence de 11 paires de nucléotides inversée de celle-ci.

3. Séquence d'ADN contenant une séquence d'ADN de la revendication 1 et ayant des séquences de reconnaissance d'enzymes de restriction ou des extrémités de clivage d'enzymes de restriction des deux côtés de la séquence de la revendication 1.

4. Séquence d'ADN contenant une séquence d'ADN de la revendication 2 et ayant des séquences de reconnaissance d'enzymes de restriction ou des extrémités de clivage d'enzymes de restriction des deux côtés de la séquence de la revendication 2.

5. Séquence d'ADN selon la revendication 4, qui a une séquence de bases telle que décrite ci-dessous SEQ ID No : 1:

AACGAATTC ATC GAG GGT CGC GACCCTCGAT GGATCCGTT

6. Séquence d'ADN selon la revendication 4, qui a une séquence de bases telle que décrite ci-dessous SEQ ID No 2 :

AACGAATTC ATC GAG GGT CGC GACCCTCGAT GAATTCGTT

7. Procédé pour préparer une séquence d'ADN contenant un gène de fusion qui comprend les étapes consistant à insérer un ADN linker (lieur) du facteur Xa selon l'une quelconque des revendications 3 à 6 dans le gène de structure d'un vecteur d'expression et à ligaturer un nouveau gène étranger avec le site de clivage de l'enzyme de restriction NruI dudit ADN linker.

8. Procédé pour préparer une séquence d'ADN contenant un gène de fusion selon la revendication 7, dans lequel un gène de fusion obtenu en ligaturant au moins deux gènes étrangers en tandem via l'ADN linker du facteur Xa est utilisé comme ledit nouveau gène étranger.

9. Vecteur d'expression portant un gène de fusion dans lequel un ADN linker du facteur Xa selon l'une quelconque des revendications 3 à 6 est contenu dans le gène de structure du vecteur d'expression.

10. Vecteur d'expression selon la revendication 9 dans lequel l'ADN linker du facteur Xa est inséré dans le gène 10 d'un vecteur contenant le promoteur dérivé du phage T7 et le gène 10 de T7 et un nouveau gène étranger est ligaturé avec le site de clivage de l'enzyme de restriction NruI dudit ADN linker.

11. Vecteur d'expression selon la revendication 10, dans lequel un gène de fusion qui peut être obtenu en ligaturant au moins deux gènes étrangers en tandem via l'ADN linker du facteur Xa est utilisé comme ledit nouveau gène étranger.

12. Vecteur d'expression selon l'une quelconque des revendications 9 à 11, dans lequel ledit gène étranger est la rhodopsine de poulpe ou un fragment de celle-ci.

13. Organisme hôte contenant un vecteur d'expression selon l'une quelconque des revendications 9 à 12.

14. Organisme hôte selon la revendication 13, où ledit organisme est E. coli.
